(19) Europäisches Patentamt
European Patent Office
Office européen des brevets

(11) **EP 4 745 160 A1**

(12) **EUROPEAN PATENT APPLICATION**
published in accordance with Art. 153(4) EPC

(43) Date of publication:
**20.05.2026 Bulletin 2026/21**

(21) Application number: 24853756.5

(22) Date of filing: **13.08.2024**

(51) International Patent Classification (IPC):
$C07K\ 16/40^{(2006.01)}$  $A61K\ 39/395^{(2006.01)}$
$C07K\ 16/38^{(2006.01)}$  $A61P\ 3/06^{(2006.01)}$
$A61P\ 9/00^{(2006.01)}$

(52) Cooperative Patent Classification (CPC):
A61K 39/395; A61P 3/06; A61P 9/00; C07K 16/38;
C07K 16/40

(86) International application number:
**PCT/CN2024/111651**

(87) International publication number:
**WO 2025/036347 (20.02.2025 Gazette 2025/08)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **14.08.2023 CN 202311023490**

(71) Applicants:
• **Salubris (Chengdu) Biotech Co., Ltd.
Chengdu, Sichuan 610041 (CN)**
• **Shenzhen Salubris Pharmaceuticals Co., Ltd.
Shenzhen, Guangdong 518017 (CN)**

(72) Inventors:
• **DENG, Chongyang
Chengdu, Sichuan 610041 (CN)**
• **WANG, Anqi
Chengdu, Sichuan 610041 (CN)**
• **HE, Shiying
Shenzhen, Guangdong 518017 (CN)**
• **TAN, Jin
Shenzhen, Guangdong 518017 (CN)**

(74) Representative: **Groth & Co. KB
P.O. Box 6107
102 32 Stockholm (SE)**

(54) **METHOD FOR TREATING OR PREVENTING CHOLESTEROL-RELATED DISEASES USING PCSK9 INHIBITOR**

(57) The present invention relates to the field of medicines, and particularly relates to a method for treating or preventing cholesterol-related diseases using a PCSK9 inhibitor. The method comprises: administering 35-420 mg of a PCSK9 antibody to a subject, thus not only reducing the frequency of drug administration, but also prolonging the duration of drug action.

EP 4 745 160 A1

## Description

### TECHNICAL FIELD

The present invention relates to the field of medicines, and particularly relates to a method for treating or preventing cholesterol-related diseases using a PCSK9 inhibitor.

### BACKGROUND

Hyperlipidemia is a group of heterogeneous disorders characterized by excessive lipids in the blood (such as cholesterol, phospholipids, and triglycerides), while hypercholesterolemia refers to elevated levels of cholesterol in the blood. Primary hypercholesterolemia is generally caused by genetic factors (monogenic or polygenic) and environmental factors (such as diet and lifestyle). The therapeutic goal of treating primary hypercholesterolemia is to reduce the level of low-density lipoprotein cholesterol (LDL-C) in the blood, since elevated LDL-C is a significant risk factor for atherosclerotic cardiovascular disease (ASCVD), lowering LDL-C levels can significantly reduce the incidence and mortality risk of ASCVD. Although various oral lipid-lowering drugs are currently available on the market, less than 30% of patients at high risk of ASCVD reach the recommended target LDL-C level (< 2.6 mmol/L), and less than 10% of patients at very high risk reach the target LDL-C level (< 1.8 mmol/L). With the increasing incidence and prevalence of primary hypercholesterolemia, there remains a substantial unmet medical need for improved efficacy, safety, and convenience of lipid-lowering therapies.

The recycling of low-density lipoprotein receptor (LDLR) on a surface of hepatocytes plays a crucial role in maintaining cellular and systemic cholesterol homeostasis by regulating plasma LDL-C levels. Studies have shown that proprotein convertase subtilisin/kexin type 9 (PCSK9) plays an important role in the recycling and regulation of LDLR. PCSK9 is a member of the subtilisin family of serine proteases, and is primarily expressed in the liver, kidney, and intestine. After secretion, it reduces an amount of LDLR on the surface of hepatocytes by binding to LDLR on the surface of hepatocytes and targeting LDLR to lysosomal degradation. A reduction in hepatic LDLR leads to an increase in circulating LDL-C levels. Therefore, PCSK9 may represent a novel therapeutic inhibitory target in patients with primary hypercholesterolemia (heterozygous familial and non-familial), mixed dyslipidemia, and homozygous familial hypercholesterolemia.

Currently, Patent WO2021243002 discloses PCSK9 inhibitors and methods of use thereof to treat cholesterol-related disorders. However, the patent mainly focuses on minors under 18 years of age and requires injection of Evolocumab at a dose of 350-500 mg/L every two weeks or every four weeks, which involves relatively frequent dosing and high dosage, and a relatively short duration of therapeutic effects.

### SUMMARY

To address the deficiencies of the prior art, the present invention provides a method for treating or preventing cholesterol-related diseases using a PCSK9 inhibitor. The method not only reduces the frequency of drug administration and dosage, but also prolongs the duration of drug efficacy.

The above objectives of the present invention are achieved by the following technical solutions.

In a first aspect, the present invention provides a method for treating cholesterol-related diseases, including: administering 35-420 mg of a PCSK9 antibody to a subject, where a heavy chain variable region of the PCSK9 antibody includes an HCDR1 as shown in SEQ ID NO: 1, an HCDR2 as shown in SEQ ID NO: 2, and an HCDR3 as shown in SEQ ID NO: 3; and a light chain variable region of the PCSK9 antibody includes an LCDR1 as shown in SEQ ID NO:7, an LCDR2 as shown in SEQ ID NO: 8, and an LCDR3 as shown in SEQ ID NO: 9.

A heavy chain variable region of the PCSK9 antibody is as shown in SEQ ID NO: 13, and the light chain variable region is as shown in SEQ ID NO: 14.

As a preferred technical solution of the present invention, the PCSK9 antibody is administered at any value within a range of 35-420 mg, for example, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, 410 mg, or 420 mg.

As a preferred technical solution of the present invention, 70 mg of the PCSK9 antibody is administered to the subject.

As a preferred technical solution of the present invention, 140 mg of the PCSK9 antibody is administered to the subject.

As a preferred technical solution of the present invention, 280 mg of the PCSK9 antibody is administered to the subject.

As a preferred technical solution of the present invention, 420 mg of the PCSK9 antibody is administered to the subject.

As a preferred technical solution of the present invention, the PCSK9 antibody is administered to the subject once every week, once every two weeks, once every three weeks, once every four weeks, once every five weeks, once every six weeks, once every seven weeks, once every eight weeks, once every nine weeks, once every ten weeks, or once every twelve weeks, or twice every two weeks, twice every three weeks, twice every four weeks, twice every five weeks, twice every six weeks, twice every seven weeks, twice every eight weeks, twice every nine weeks, twice every ten weeks, or twice every twelve weeks.

As a preferred technical solution of the present invention, 70 mg of the PCSK9 antibody is administered to the subject once every week or once every two weeks.

As a preferred technical solution of the present invention, 140 mg of the PCSK9 antibody is administered to the subject once every two weeks, once every four weeks, once every six weeks, or once every eight weeks, and preferably, 140 mg of the PCSK9 antibody is administered once every four weeks.

As a preferred technical solution of the present invention, 280 mg of the PCSK9 antibody is administered to the subject once every two weeks, once every four weeks, once every six weeks, or once every eight weeks.

As a preferred technical solution of the present invention, 420 mg of the PCSK9 antibody is administered to the subject once every four weeks, once every six weeks, once every eight weeks or once every twelve weeks, and preferably, 420 mg of the PCSK9 antibody is administered once every six weeks, or every eight weeks.

As a preferred technical solution of the present invention, an administration route of the PCSK9 antibody is subcutaneous injection or intramuscular injection.

As a preferred technical solution of the present invention, 1-16 weeks after 70-420 mg of the PCSK9 antibody is administered to the subject, an area under the plasma concentration-time curve from zero to the time of the last quantifiable concentration ($AUC_{0-t}$) in the subject is at least greater than 5,000,000 ng•h/mL. For example, 4 weeks after 140 mg of the PCSK9 antibody is administered to the subject, the area under the plasma concentration-time curve from zero to the time of the last quantifiable concentration ($AUC_{0-t}$) in the subject is greater than 5,000,000 ng•h/mL; and for another example, 8 weeks after 420 mg of the PCSK9 antibody is administered to the subject, the area under the plasma concentration-time curve from zero to the time of the last quantifiable concentration ($AUC_{0-t}$) in the subject is greater than 30,000,000 ng•h/mL.

As a preferred technical solution of the present invention, after 140-420 mg of the PCSK9 antibody is administered to the subject, a reduction of low-density lipoprotein cholesterol (LDL-C) by at least 50% is maintained for 25-60 days; where a duration of the reduction is any value in a range of 25-60 days, for example, after 140 mg of the PCSK9 antibody is administered to the subject, a reduction of LDL-C by at least 50% is 27 days, 29 days, 31 days, 33 days, 35 days, 37 days, 39 days, 41 days, 43 days, 45 days, 47 days, 49 days, 51 days, 53 days, 55 days, 57 days, or 59 days. For another example, after 420 mg of the PCSK9 antibody is administered to the subject, a reduction of LDL-C by at least 50% is 27 days, 29 days, 31 days, 33 days, 35 days, 37 days, 39 days, 41 days, 43 days, 45 days, 47 days, 49 days, 51 days, 53 days, 55 days, 57 days, or 59 days.

In a second aspect, the present invention provides a method for treating or preventing cholesterol-related diseases, including:

administering a clinical dose of a statin drug and 35-420 mg of a PCSK9 antibody to a subject;
where a heavy chain variable region of the PCSK9 antibody includes an HCDR1 as shown in SEQ ID NO: 1, an HCDR2 as shown in SEQ ID NO: 2, and an HCDR3 as shown in SEQ ID NO: 3; and a light chain variable region of the PCSK9 antibody includes an LCDR1 as shown in SEQ ID NO: 7, an LCDR2 as shown in SEQ ID NO: 8, and an LCDR3 as shown in SEQ ID NO: 9.

As a preferred technical solution of the present invention, the PCSK9 antibody is administered at any value within a range of 35-420 mg, for example, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, 410 mg, or 420 mg.

As a preferred technical solution of the present invention, 70 mg of the PCSK9 antibody is administered to the subject.

As a preferred technical solution of the present invention, 140 mg of the PCSK9 antibody is administered to the subject.

As a preferred technical solution of the present invention, 280 mg of the PCSK9 antibody is administered to the subject.

As a preferred technical solution of the present invention, 420 mg of the PCSK9 antibody is administered to the subject.

As a preferred technical solution of the present invention, the PCSK9 antibody is administered to the subject once every week, once every two weeks, once every three weeks, once every four weeks, once every five weeks, once every six weeks, once every seven weeks, once every eight weeks, once every nine weeks, once every ten weeks, or once every twelve weeks, or twice every two weeks, twice every three weeks, twice every four weeks, twice every five weeks, twice every six weeks, twice every seven weeks, twice every eight weeks, twice every nine weeks, twice every ten weeks, or twice every twelve weeks.

As a preferred technical solution of the present invention, 70 mg of the PCSK9 antibody is administered to the subject once every week or once every two weeks.

As a preferred technical solution of the present invention, 140 mg of the PCSK9 antibody is administered to the subject once every two weeks, once every four weeks, once every six weeks, or once every eight weeks.

As a preferred technical solution of the present invention, 280 mg of the PCSK9 antibody is administered to the subject once every two weeks, once every four weeks, once every six weeks, or once every eight weeks.

As a preferred technical solution of the present invention, 420 mg of the PCSK9 antibody is administered to the subject once every four weeks, once every six weeks, once every eight weeks, or once every twelve weeks.

As a preferred technical solution of the present invention, the PCSK9 antibody is administered by subcutaneous injection or intramuscular injection, and the statin drug is administered orally.

As a preferred technical solution of the present invention, the statin drug is selected from atorvastatin, cerivastatin, fluvastatin, lovastatin, mevastatin, pitavastatin, pravastatin, rosuvastatin, and simvastatin; and the statin drug may be appropriately adjusted according to the clinical dose.

As a preferred technical solution of the present invention, the clinical dose of atorvastatin is 10-80 mg, and preferably 20 mg or 30 mg.

Specifically, the statin drug may further include 40 mg atorvastatin, 80 mg atorvastatin, 20 mg rosuvastatin, 40 mg rosuvastatin, 40 mg simvastatin, or 80 mg simvastatin.

As a preferred technical solution of the present invention, 1-16 weeks after 70-420 mg of the PCSK9 antibody is administered to the subject, an area under the plasma concentration-time curve from zero to the time of the last quantifiable concentration ($AUC_{0-inf}$) in the subject is at least greater than 4,000,000 ng•h/mL. For example, 4 weeks after 140 mg of the PCSK9 antibody is administered to the subject, the area under the plasma concentration-time curve from zero to the time of the last quantifiable concentration ($AUC_{0-inf}$) in the subject is at least greater than 4,000,000 ng•h/mL. For example, 8 weeks after 420 mg of the PCSK9 antibody is administered to the subject, the area under the plasma concentration-time curve from zero to the time of the last quantifiable concentration ($AUC_{0-inf}$) in the subject is at least greater than 10,000,000 ng•h/mL.

The antibody of the present invention is more preferably the 18.156.8 antibody as disclosed in Chinese Patent Application No. CN201710816808.0, in which "18.156.8 (hIgG4)" refers to the 18.156.8 antibody with a human IgG4 isotype constant region.

Compared with the prior art, the present invention has the following advantages and beneficial effects: administering the PCSK9 antibody inhibitor of the present invention to a subject not only reduces the frequency of drug administration, but also significantly lowers LDL-C levels, and the duration of LDL-C reduction is prolonged with increasing dose.

**Definition**

The term "include/including, comprise/comprising" as used herein is intended to mean the phrase "including (but not limited to)", and is interchangeable therewith, unless otherwise explicitly stated in the context.

The terms "proprotein convertase subtilisin/kexin type 9 (PCSK9)", "PCSK9", or "NARC-1" refer to a naturally occurring human proprotein convertase belonging to the proteinase K subfamily of secreted subtilisin family. PCSK9 is synthesized as a plasminogen, undergoes autocatalytic intramolecular processing in the endoplasmic reticulum, and is believed to function as a proprotein convertase. As used herein, the term refers to any naturally occurring PCSK9, preferably derived from any vertebrate source (including mammals such as primates (e.g., humans) and rodents (e.g., mice and rats).

The term "PCSK9" encompasses "full-length" unprocessed PCSK9, as well as any form of PCSK9 or any fragment thereof produced by intracellular processing. The term further includes naturally occurring variants of PCSK9, for example, splice variants, derivative variants, substitution variants, deletion variants, and/or insertion variants or allelic variants, such as the mutants D374Y, S127R, and F216L.

The term "antibody" includes any immunoglobulin, monoclonal antibody, polyclonal antibody, multispecific antibody, or bispecific (bivalent) antibody capable of binding to a specific antigen. A naturally occurring intact antibody consists of two heavy chains and two light chains. Each heavy chain consists of one variable region (VH) and three constant regions (CH1, CH2, and CH3), with a hinge region located between CH1 and CH2; and each light chain consists of one variable region (VL) and one constant region (CL). Mammalian heavy chains may be classified into α, δ, ε, γ, or μ, and mammalian light chains may be classified into λ or κ. Antibodies are "Y"-shaped. A stem of the "Y"-shaped structure is formed by the CH2 and CH3 regions of the two heavy chains, which are linked by disulfide bonds in the hinge region. Each arm of the "Y"-shaped structure includes the variable region and CH1 of one heavy chain, which is linked to the variable region and CL of one light chain by disulfide bonds. The variable regions of the heavy chain and light chain determine the binding of the antibody to the antigen. A variable region of each chain contains three hypervariable regions (HVRs), also known as complementarity-determining regions (CDRs), where the CDR of the light chain (L) contains LCDR1, LCDR2, and LCDR3, and the CDR of the heavy chain (H) contains HCDR1, HCDR2, and HCDR3. CDR boundaries of an antibody or an antigen-binding fragment thereof disclosed in the present invention may be defined or identified by the Kabat, Chothia, IMGT, AbM, or Contact numbering systems/methods.

Specifically, each heavy-chain CDR and/or each light-chain CDR may be defined according to the Kabat definition, the Chothia definition, a combination of the Kabat and Chothia definitions, the IMGT definition, the AbM definition, or a CDR contact definition. Preferably, each CDR is defined according to the Kabat definition, the Chothia definition, or a combination of both of them.

PCSK9 inhibitor refers to a molecule or therapy that inhibits PCSK9 activity, thereby reducing levels of LDL--C (and/or other lipids, such as non-HDL-C, ApoB, Lp(a)). For example, this may include neutralizing antibodies against PCSK9 and antisense molecules against PCSK9. PCSK9 inhibitor therapy refers to a method using the PCSK9 inhibitor.

The term "LDL-C" refers to low-density lipoprotein cholesterol. The term "HDL-C" refers to high-density lipoprotein cholesterol. LDL and HDL belong to the five major classes of lipoproteins: chylomicrons, very-low-density lipoproteins (VLDL), intermediate-density lipoproteins (IDL), low-density lipoproteins (LDL), and high-density lipoproteins (HDL) (in order from the largest particles to the most dense/smallest particles). LDL (also known as "bad" particle-containing cholesterol) is capable of transporting lipid/sterol molecules, such as cholesterol (that is, LDL-C), to the arterial wall, attracting macrophages and thus inducing atherosclerosis. In contrast, HDL (also known as "good" particle-containing cholesterol) is capable of removing lipid molecules, such as cholesterol (that is, HDL-C) from macrophages in the arterial wall. Therefore, high levels of LDL-C represent a major risk factor for cardiovascular diseases (CVD), such as peripheral artery disease, coronary artery disease (CAD, such as angina pectoris, myocardial infarction (commonly known as heart attack), hyperlipidemia, hypercholesterolemia, and hypertriglyceridemia), atherosclerosis, stroke, hypertensive heart disease, rheumatic heart disease, cardiomyopathy, arrhythmia, congenital heart disease, valvular heart disease, myocarditis, aneurysm, peripheral artery disease, obesity, hepatobiliary diseases, nephrotic syndrome, hypothyroidism, and venous thrombosis.

The term "treatment" encompasses alleviating at least one symptom or other embodiments of a disorder, or reducing the severity of a disease. A PCSK9 inhibitor and/or one or more other LDL-cholesterol-lowering therapies do not need to achieve complete cure or eradication of every symptom or manifestation of the disease to be considered a viable therapeutic agent. As recognized in the relevant fields, a drug used as a therapeutic agent may reduce the severity of a given disease state without necessarily eliminating every manifestation of the disease to be considered a useful therapeutic agent. In some embodiments, it is sufficient to merely reduce the impact of a disease (for example, by reducing the frequency or severity of its symptoms, by increasing the effectiveness of another therapy, or by producing another beneficial effect), or to reduce the likelihood of occurrence or worsening of the disease in a subject. Some embodiments of the present invention relate to methods including administering a PCSK9 inhibitor (for example, an anti-PCSK9 antibody or an interfering RNA) to a subject, where an amount and timing of administration of the inhibitor are sufficient to induce a sustained improvement compared to a baseline of an indicator reflecting the severity of a particular disorder.

The term "prevention" encompasses preventing at least one symptom or other embodiments of a disorder, etc. According to the present invention, treatment involving the prophylactic administration of a PCSK9 inhibitor and/or one or more other LDL-cholesterol-lowering therapies does not need to be completely effective in preventing the onset of conditions to be considered a viable preventive agent. In some embodiments, it is sufficient to merely reduce the likelihood of occurrence or worsening of a disease in a subject. In some embodiments, progression of disease symptoms is delayed by the treatment methods provided by the present invention.

The term "cholesterol-related diseases" include, but are not limited to, hyperlipoproteinemia, hyperlipidemia; dyslipidemia (for example, elevated total cholesterol, elevated LDL, elevated triglycerides, elevated VLDL and/or reduced HDL); hypercholesterolemia, mixed hyperlipidemia, heart disease, stroke, coronary heart disease, atherosclerosis, peripheral vascular disease, claudication, type 2 diabetes, hypertension, cardiovascular diseases or symptoms, inflammation or autoimmune diseases. Methods for identifying and/or diagnosing the above diseases or symptoms are known in the art.

The term "subject" includes any human or non-human animals. "Non-human animals" include all vertebrates, such as mammals (including but not limited to domesticated animals (such as cattle, sheep, cats, dogs, and horses), primates (such as monkeys), rabbits, and rodents (such as mice and rats)), and non-mammals (such as poultry, amphibians, and reptiles). In some embodiments, the subject is a human.

The term "therapeutically effective amount" or "effective dose" refers to a dose or concentration that, when administered at a required dose and for a required duration of time, effectively prevents or ameliorates symptoms associated with a disease or condition and/or reduces the severity of the disease or condition. A therapeutically effective amount of a formulation, an antibody or an antigen-binding fragment thereof, or a composition of the present invention may vary depending on various factors, such as disease state, age, sex, and body weight of an individual, and the ability of the antibody or an antibody portion to elicit the desired response in the individual. A therapeutically effective amount may also be considered any amount of a formulation, an antibody or an antigen-binding fragment thereof, or a composition in which any toxic or harmful effects are outweighed by the therapeutic benefits.

## DETAILED DESCRIPTIONS OF THE EMBODIMENTS

The present invention will be described in further detail below in conjunction with specific embodiments, but the implementation modes of the present invention are not limited thereto.

## Example 1 Preparation of PCSK9 antibody

A PCSK9 antibody (Antibody A) provided by the present invention was prepared with reference to the preparation method of Antibody 18.156.8 disclosed in Chinese Patent Application No. CN201710816808.0. The preparation method disclosed in the above patent is incorporated herein by reference in their entirety.

Table 1 shows the amino acid sequences and nucleic acid sequences of Antibody A.

| | (CDR1) SEQ ID NO: 1 | (CDR2) SEQ ID NO: 2 | (CDR3) SEQ ID NO: 3 |
|---|---|---|---|
| C-D-R-V-H | SYGMH | VIWYDGTNKYYADS VKG | EKGLD |
| | SEQ ID NO: 4 | SEQ ID NO: 5 | SEQ ID NO: 6 |
| | AGC TAT GGCATG CAC | GTT ATA TGG TATGAT GGA ACTAATAAA TAC TAT GCAGAC TCC GTG AAGGGC | GAG AAG GGG CTGGAC |
| | (CDR1) SEQ ID NO: 7 | (CDR2) SEQ ID NO: 8 | (CDR3) SEQ ID NO:9 |
| C-D-R-V-L | KSSQSVLYSSTNKNYLV | WASTRES | QQYYSTPWT |
| | SEQ ID NO: 10 | SEQ ID NO:11 | SEQ ID NO: 12 |
| | AAG TCC AGCCAG AGT GTTTTA TAC AGCTCC ACC AATAAG AAC TACTTA GTT | TGG GCA TCT ACCCGG GAA TCC | CAG CAA TAT TATAGT ACT CCG TGGACG |
| V-H | SEQ ID NO: 13 | | |
| | QVQLVESGGGVVQPGRSLRLSCAASGFTFSSYGMHWVRQAPGKGLEWMAVIWYDGTNK YYADSVKGRFTISRDNSKNTVYLQMNSLRAEDTAVYYCAREKGLDWGQGTLVTVSS | | |
| V-L | SEQ ID NO: 14 | | |
| | DIVMTQSPDSLAVSLGERATINCKSSQSVLYSSTNKNYLVWYQQKPGQPPKLLIYWASTRE SGVPDRFSGSGSGTDFTLTISSLQAEDVAVYYCQQYYSTPWTFGQGTKVEIK | | |

EP 4 745 160 A1

7

**Comparative Example 1 Repatha® (Evolocumab)** was purchased from Amgen Inc., with a specification of 140 mg/ml.

**Example 2**

This trial was a single-center, single-dose, dose-escalation, randomized (with the 35 mg being non-randomized), double-blind, placebo/Repatha®-controlled Phase I clinical study conducted in healthy volunteers. A total of five dose groups were established, namely 35 mg, 70 mg, 140 mg, 280 mg, and 420 mg. The study proceeded sequentially starting from the lowest dose of 35 mg. When the dose was tolerated within 7 days (35 mg and 70 mg groups)/14 days (140 mg, 280 mg, and 420 mg groups) after dosing, as determined by the investigator, enrollment into the next dose group was permitted. Each volunteer received only one assigned dose.

A maximum of 34 healthy volunteers, both male and female, of both sexes were planned to be enrolled. The study design for each dose group was as follows:

35 mg: two volunteers were planned to be enrolled, and both received a single subcutaneous injection of Antibody A; and the volunteers were required to remain hospitalized for observation until Day 6 after dosing and were followed up until Day 29 after dosing. This dose group was evaluated only for tolerability, safety, pharmacodynamics (PD), and immunogenicity, and pharmacokinetic (PK) characteristics were not evaluated.

70 mg: Four volunteers were planned to be enrolled in this group and were randomized at a ratio of 3:1 to receive either Antibody A or placebo via a single subcutaneous injection; and the volunteers were required to remain hospitalized for observation until Day 6 after dosing and were followed up until Day 29 after dosing. This group of volunteers was evaluated for tolerability, safety, PK, PD, and immunogenicity.

140 mg: 12 volunteers were planned to be enrolled in this group and were randomized at a ratio of 1:1 to receive either Antibody A or Repatha® via a single subcutaneous injection; and the volunteers were required to remain hospitalized for observation until Day 3 after dosing and were followed up until Day 113 after dosing. Volunteers in this dose group were evaluated for tolerability, safety, PK, PD, and immunogenicity.

280 mg: Four volunteers were planned to be enrolled in this group and were randomized at a ratio of 3:1 to receive either Antibody A or placebo via a single subcutaneous injection; and the volunteers were required to remain hospitalized for observation until Day 3 after dosing and were followed up until Day 29 after dosing. This dose group was evaluated only for tolerability, safety, PD, and immunogenicity, and PK characteristics were not evaluated.

420 mg: 12 volunteers were planned to be enrolled in this group and were randomized at a ratio of 1:1 to receive either Antibody A or Repatha® via a single subcutaneous injection; and the volunteers were required to remain hospitalized for observation until Day 3 after dosing and were followed up until Day 113 after dosing. Volunteers in this dose group were evaluated for tolerability, safety, PK, PD, and immunogenicity.

Number of volunteers (planned and analyzed)

A maximum of 34 healthy volunteers were planned to be enrolled. A total of 34 healthy volunteers actually participated in the study, including 20 subjects in the investigational drug group, 2 subjects in the placebo group, and 12 subjects in the positive control group. The distribution of volunteers is shown below:

Table 2 Distribution of volunteers

|  | 35 mg | 70 mg | 140 mg | 280 mg | 420 mg | Total |
|---|---|---|---|---|---|---|
| Planned enrollment | 2/0 | 3/1 | 6/6 | 3/1 | 6/6 | 34 |
| Randomized | 2/0 | 3/1 | 6/6 | 3/1 | 6/6 | 34 |
| Received dosing | 2/0 | 3/1 | 6/6 | 3/1 | 6/6 | 34 |
| Study completion | 2/0 | 3/1 | 6/6 | 3/1 | 6/6 | 34 |
| Exited the study | 0/0 | 0/0 | 0/0 | 0/0 | 0/0 | 0 |
| Note: The number of subjects is indicated as Antibody A or Repatha®. | | | | | | |

Inclusion criteria: Volunteers were required to meet all of the following criteria to be eligible for enrollment:

(1) healthy adults aged 18-45 years (including the threshold), male or female;
(2) body weight $\geq$ 50.0 kg for male volunteers and $\geq$ 45.0 kg for female volunteers, with a body mass index (BMI) between 19.0-26.0 kg/m$^2$ (inclusive); and

(3) voluntarily signed a written informed consent form.

Exclusion criteria: Volunteers meeting one or more of the following criteria are excluded:

(1) individuals with a history of or currently suffering from any clinically significant disease of the circulatory, endocrine, nervous, digestive, respiratory, urogenital, hematological, immunological, psychiatric systems, or metabolic abnormalities, or any other disease that could interfere with study results;
(2) Individuals with a history of headaches or migraines;
(3) Individuals with a history of recurrent nausea or vomiting;
(4) Individuals with a history of allergy to medications, food, or other substances;
(5) individuals who have undergone surgery within 4 weeks prior to the study, or who have planned surgery during the study period, or who have undergone surgery that could affect drug absorption, distribution, metabolism, or excretion;
(6) Individuals who have taken any medications or health supplements (including traditional Chinese medicine) within 14 days prior to the study;
(7) individuals who have used any drugs that inhibit or induce hepatic drug metabolism within 30 days prior to the study (such as inducers-barbiturates, carbamazepine, phenytoin, glucocorticoids, omeprazole; inhibitors-selective serotonin reuptake inhibitors (SSRIs), cimetidine, diltiazem, macrolides, nitroimidazoles, sedative-hypnotics, verapamil, fluoroquinolones, antihistamines, clarithromycin, nefazodone, ritonavir, and atazanavir);
(8) individuals who have participated in any clinical trial and taken any investigational drugs within 3 months prior to the study, or who have received any treatment targeting PCSK9;
(9) individuals who have donated blood or experienced significant blood loss ($\geq$ 200 mL), received blood transfusion, or used blood products within 3 months prior to enrollment;
(10) pregnant or breastfeeding women;
(11) individuals who have had unprotected sexual activity within 2 weeks prior to screening, and who have planned to conceive and were unwilling to use or more non-drug contraceptive methods during the study period or within 6 months after the end of the study;
(12) individuals with special dietary requirements who cannot adhere to standardized diet;
(13) individuals who consumed an excessive amount of tea, coffee, or caffeinated beverages daily (more than 8 cups/day, 1 cup = 250 mL) within 3 months prior to the study;
(14) heavy smokers or individuals who smoked more than 5 cigarettes per day within 3 months prior to the study, or individuals who cannot stop using any tobacco products during the study;
(15) individuals with a history of alcohol abuse (defined as men consuming 5 units or more of alcohol, or women consuming 4 units or more within 2 hours), or individuals who frequently consumed alcohol, that is, consuming more than 14 units of alcohol per week within 6 months prior to the study (1 unit = 360 mL of beer or 45 mL of spirits of 40% alcohol content or 150 mL of wine), or individuals who cannot stop using any alcohol-containing products during the study;
(16) individuals with a history of drug use or who have used soft drugs (e.g., marijuana) within 3 months prior to the study, or hard drugs (e.g., cocaine, phencyclidine) within 1 year prior to the study;
(17) individuals with abnormal vital signs (systolic blood pressure <90 mmHg or >140 mmHg, diastolic blood pressure <50 mmHg or >90 mmHg; heart rate <50 bpm or >100 bpm), or clinically significant abnormalities in physical examination, electrocardiogram, or laboratory tests (as determined by the clinical research physician);
(18) individuals unable to tolerate venous blood sampling; and
(19) individuals whose LDL-C level was < 1.8 mmol/L or > 4.14 mmol/L during screening; and
(20) volunteers who were unable to complete the study for other reasons, or the researchers deemed inappropriate for enrollment by the investigator.

Primary objective: To evaluate the safety and tolerability of a single dose of Antibody A at dose levels ranging from 35 mg to 420 mg in healthy Chinese adult subjects.
Secondary objectives: To evaluate the pharmacokinetic and pharmacodynamic characteristics of a single dose of Antibody A at dose levels ranging from 35 mg to 420 mg in healthy Chinese adult subjects, and to preliminarily assess the immunogenicity after the single dosing.
Investigational drug, dosage, and administration route:

Name: Recombinant fully human anti-PCSK9 monoclonal antibody injection (Antibody A)
Specification: 140 mg/mL (1 mL)
Dosage: 35 mg, 70 mg, 140 mg, 280 mg, and 420 mg
Administration route: After fasting overnight for at least 10 hours, volunteers received a standardized breakfast on Day 1, followed by a single subcutaneous injection administered into the abdominal region.

Study duration: A study duration for each volunteer included: a screening period of 1-21 days; an inpatient observation period of 3 days (140 mg, 280 mg, and 420 mg groups)/6 days (35 mg and 70 mg groups); and a follow-up period of 23 days (35 mg, 70 mg, and 140 mg groups)/110 days (140 mg and 420 mg groups).

Positive control:

Name: Evolocumab injection (Repatha®)
Specification: 140 mg/mL (1 mL)
Dosage: 140 mg and 420 mg
Administration route: After fasting overnight for at least 10 hours, volunteers received a standardized breakfast on Day 1, followed by a single subcutaneous injection administered into the abdominal region.

Placebo:

Name: Simulant of recombinant fully human anti-PCSK9 monoclonal antibody injection (Antibody A)
Administration route: After fasting overnight for at least 10 hours, volunteers received a standardized breakfast on Day 1, followed by a single subcutaneous injection administered into the abdominal region.

Evaluation criteria:

Pharmacokinetics: concentrations of the investigational drugs (Antibody A and Repatha®) in serum.
Pharmacodynamic variables:
Serum free PCSK9 concentration;
Concentrations of lipid profile indicators (total cholesterol, triglycerides, high-density lipoprotein cholesterol, low-density lipoprotein cholesterol, small dense low-density lipoprotein cholesterol, non-high-density lipoprotein cholesterol, apolipoprotein A1, apolipoprotein B, and lipoprotein (a);
Safety variables: adverse events, vital signs, physical examinations, 12-lead electrocardiograms, clinical laboratory tests (complete blood count, urinalysis, blood biochemistry, lipid profiles, and coagulation function), and anti-drug antibodies /neutralizing antibodies.

Summary overview:

Safety conclusions: No maximum tolerated dose (MTD) of Antibody A was observed in this study, and no serious adverse events (SAEs) occurred. A total of 81 treatment-emergent adverse events (TEAEs) were reported by 85% of the volunteers. The incidence of TEAEs for Antibody A was comparable to that of Repatha®, with no dose-related trend observed. The most common TEAE was elevated blood triglycerides, all of which were considered possibly unrelated to the investigational product; and all adverse drug reactions (ADRs) observed with Antibody A were previously reported ADRs for Repatha®, and no unexpected adverse reactions were identified. In addition, no significant changes in anti-drug antibodies (ADA) were observed before or after dosing with either Antibody A or Repatha®, and all neutralizing antibody (Nab) results were negative. In summary, Antibody A demonstrated good safety and tolerability after a single dosing.

Pharmacokinetics results

Since only three volunteers were included in the 70 mg dose group, descriptive statistical analysis was not performed for this group. Main pharmacokinetic parameters of Antibody A at doses of 140 mg and 420 mg, and those of Repatha® at doses of 140 mg and 420 mg are shown in the table below:

Table 3 Main pharmacokinetic parameters (PKPS) for each dose group

| - | Antibody A 140 mg | Repatha® 140 mg | Antibody A 420mg | Repatha® 420 mg |
|---|---|---|---|---|
| Number of cases (missing) | 6 (0) | 6 (0) | 6 (0) | 6 (0) |

(continued)

| - | | Antibody A 140 mg | Repatha® 140 mg | Antibody A 420mg | Repatha® 420 mg |
|---|---|---|---|---|---|
| $T_{max}$(h) | Mean (Q1, Q3) | 99.591 (72.000, 120.005) | 76.000 (71.999, 72.000) | 151.876 (119.763,239.841) | 84.013(72.001,72.031) |
| $t_{1/2Z}$(h) | Mean (Standard deviation) | 143.354 (16.795) | 102.822 (33.630) | 205.873 (22.769) | 286.443 (42.165) |
| Cmax (ng·mL$^{-1}$) | Mean (Standard deviation) | 16255.234 (5009.795) | 11413.818 (1820.051) | 45309.854 (13958.533) | 43553.517 (11736.700) |
| AUC0-t (ng·h·mL$^{-1}$) | Mean (Standard deviation) | 5564494.890 (1802062.863) | 2536861.894 (895051.184) | 30556839.331 ( 89908281.427) | 16594938.819 (2340853.423) |
| AUC0-∞ (ng·h·mL$^{-1}$) | Mean (Standard deviation) | 5622122.736 (1809897.690) | 2754671.585 (938954.437) | 30626192.654 (8916674.639) | 17245467.810 (2267422.632) |
| CL/F (L·h$^{-1}$) | Mean (Standard deviation) | 0.028 (0.010) | 0.055 (0.015) | 0.015 (0.007) | 0.025 (0.003) |
| Vz/F(L) | Mean (Standard deviation) | 5.653 (2.249) | 7.610 (0.803) | 4.401 (1.376) | 10.200 (1.944) |

As shown in Table 3, the results of the investigational drugs show that both the inhibition rate and the duration of PCSK9 inhibition increased with increasing doses of Antibody A. At both the 140 mg and 420 mg doses, the exposure to Antibody A was significantly higher than that of Repatha®.

Pharmacodynamic results

Table 4 Changes in lipid profile indicators from baseline in different dose groups (PDPS)

| | Antibody A | | | | | Evolocumab | |
|---|---|---|---|---|---|---|---|
| | 35 mg | 70 mg | 140 mg | 280 mg | 420 mg | 140 mg | 420 mg |
| LDL-C Percentage change from baseline at the minimum value, mean (%) | -50.2 | -60.7 | -68.2 | -69.3 | -60.4 | -62.1 | -67.3 |
| Time required to reach the minimum value (days), median | 18.5 | 11.0 | 22.0 | 15.0 | 43.0 | 11.0 | 29.0 |
| Total cholesterol Percentage change from baseline at the minimum value, mean (%) | -31.5 | -43.1 | -44.8 | -42.7 | -40.6 | -36.5 | -42.7 |
| Time required to reach the minimum value (days), median | 11.5 | 15.0 | 22.0 | 15.0 | 29.0 | 11.0 | 16.5 |
| ApoB Percentage change from baseline at the minimum value, mean (%) | -53.6 | -58.5 | -54.6 | -64.7 | -51.6 | -44.2 | -57.8 |
| Time required to reach the minimum value (days), median | 18.5 | 15.0 | 22.0 | 15.0 | 36.0 | 15.0 | 25.5 |

As shown in Table 4, Antibody A significantly reduced LDL-C, and the duration of the reduction was prolonged with increasing doses. Furthermore, the duration of LDL-C, total cholesterol, and ApoB reduction of Antibody A at both the 140 mg and 420 mg doses was significantly longer than that achieved by Repatha® at the 140 mg and 420 mg doses.

In addition, comparative analysis of the duration of LDL-C reduction demonstrates that, at the 140 mg dose, Antibody A provided a significantly longer duration of LDL-C reduction than that of Repatha® of the 140 mg dose. The median time for the two drugs to reach the minimum percentage change in LDL-C from baseline was 22 days and 11 days, respectively, indicating that Antibody A at a dose of 140 mg could support dosing once every 4 weeks. In addition, in the 420 mg dose group, Antibody A provided a significantly longer duration of LDL-C reduction than that of Repatha®. The median durations to reach the minimum percentage change in LDL-C from baseline for the two drugs were 43 days and 29 days, respectively, indicating that Antibody A at a dose of 420 mg could support dosing once every 6 weeks or once every 8 weeks.

**Example 3**

This study is a multicenter, randomized, double-blind, placebo/positive-controlled study involving multiple dosing in patients with hypercholesterolemia and mixed hyperlipidemia to evaluate the safety and tolerability of Antibody A in combination with atorvastatin using different dosing regimens, to describe the pharmacokinetic characteristics in patients, and to preliminarily explore efficacy.

A total of 36 subjects with hypercholesterolemia or mixed hyperlipidemia, including both male and female, were planned to be enrolled in this study. Two enrollment pathways are designed for this study: Pathway 1: Subjects who have not previously taken atorvastatin at regular intervals and meet the screening criteria received atorvastatin monotherapy at a stable dose for a 4-week run-in period. Subjects who still meet the inclusion and exclusion criteria after the 4-week run-in period entered the double-blind treatment period. Pathway 2: Subjects who have been taking atorvastatin at regular intervals for 4 weeks or more prior to screening and met the inclusion and exclusion criteria entered the double-blind treatment period directly. After entering the double-blind treatment period, subjects from both pathways were randomized at a ratio of 5:5:5:3 to receive 140 mg of Antibody A, 420 mg of Antibody A, 420 mg of Evolocumab, and placebo, respectively.

Primary objective: To evaluate the safety, tolerability, and pharmacokinetic characteristics of multiple subcutaneous administrations of Antibody A in combination with atorvastatin using different dosing regimens.

Secondary objective: To preliminarily explore the efficacy of Antibody A in combination with atorvastatin using different dosing regimens.

Key inclusion criteria

Inclusion criteria:

1. Aged 18-65 years (inclusive 18 and 65 years old), gender unspecified.
2.

$$18.5 \text{ kg/m}^2 \leq \text{BMI} \leq 35 \text{ kg/m}^2 \ (\text{BMI} = \text{weight/height}^2).$$

3. Patients diagnosed with hypercholesterolemia and/or mixed hyperlipidemia according to the *Chinese Guidelines for the Prevention and Treatment of Dyslipidemia in Adults* (2016 Revised Edition).

4. At screening, any one of the following conditions is satisfied:

A: Pathway 1: At screening, subjects receiving any type of statin therapy must have a serum LDL-C level ≥ 2.6 mmol/L and be willing to receive stable-dose atorvastatin treatment (dosage determined by the investigator), and still meet LDL-C ≥ 2.6 mmol/L prior to randomization;

B: Pathway 2: At screening, subjects who have received stable-dose atorvastatin treatment for 28 days or more prior to screening and have a serum LDL-C level ≥ 2.6 mmol/L; and still meet LDL-C ≥ 2.6 mmol/L prior to randomization.

5.

$$\text{Fasting triglycerides} \leq 4.5 \text{ mmol/L}.$$

6. Subjects who have a thorough understanding of the study and potential adverse reactions, voluntarily participated in the study, signed a written informed consent form, and are able to comply with the study procedures of the study protocol.

Exclusion criteria

1. Patients with homozygous familial hypercholesterolemia (HoFH) or other diseases that may cause secondary elevation of LDL-C, including Cushing syndrome, nephrotic syndrome, multiple myeloma, glycogen storage disease, systemic lupus erythematosus, and acute intermittent porphyria.

2. Pathway 1: atorvastatin medication adherence <80% or >120% during the run-in period; and Pathway 2: atorvastatin medication adherence <80% or >120% within 28 days prior to screening.

3. Individuals who have a history of heart failure (Class II and IV according to heart functional classification of New York Heart Association (NYHA)), acute coronary syndrome, percutaneous coronary intervention, or other severe cardiac diseases [such as cardiogenic shock, first- or first-degree atrioventricular block, bradycardia (heart rate <50 beats/min), or other serious arrhythmias] over the past 6 months.

4. Individuals with severe cerebrovascular disease (hypertensive encephalopathy, cerebrovascular injury, stroke, transient ischemic attack, and the like), severe aortic or peripheral vascular disease, or conditions requiring surgical intervention.

5. Patients with poorly controlled hypertension (SBP >160 mmHg or DBP >100 mmHg).

6. Diabetic patients with any of the following conditions: 1) patients with type 1 diabetes; or 2) patients with type 2 diabetes with poorly controlled blood glucose (HbA1c >8.0%).

7. Individuals with active viral hepatitis (including hepatitis B or C), other severe liver disease, or hepatic dysfunction (ALT or AST > 2.5 times the upper limit of normal (ULN); total bilirubin (TBIL)> 2 times the ULN).

8. Individuals with glomerular filtration rate (eGFR) <45 ml/min/1.73 $m^2$ during screening.

9. Individuals with abnormal thyroid function (thyroid-stimulating hormone (TSH) outside the normal range of the center).

10. Individuals with creatine kinase (CK) >3 times the ULN at screening.

11. Individuals with positive HIV or syphilis test results.

12. Individuals with active malignant tumors, or a history of malignant tumors within 5 years prior to screening (including basal cell carcinoma of the skin or cervical carcinoma in situ).

13. Individuals with known allergies to the investigational drug or any of its components, or those who have a history of severe allergic reactions to other antibody-based drugs.

14. Individuals having a history of organ transplantation.

15. Individuals who have received an inactivated virus vaccine within 1 month prior to screening, or an adenoviral recombinant/mRNA vaccine within 3 months prior to screening, or planned vaccination during the study.

16. Individuals who have donated blood or experienced significant blood loss (> 400 mL) within 3 months prior to screening, or had clinical diagnosis of hypovolemia.

17. Individuals who have taken any traditional Chinese medicines or supplements affecting lipid metabolism within 30 days prior to receiving the investigational drug (e.g., fish oil >1000 mg/day, drugs or health products containing red yeast rice).

18. Individuals who have participated in other drug or device clinical trials within 3 months prior to screening, or who have received any treatment targeting proprotein convertase subtilisin//kexin type 9 (PCSK9).

19. Individuals who have a history of drug abuse or alcohol abuse within 6 months prior to screening (consuming 14 units of alcohol per week: 1 unit = 285 mL of beer, or 25 mL of spirits, or 100 mL of wine).

20. Female subjects who are breastfeeding or have positive serum pregnancy test results during the screening period or the study period.

21. Subjects who plan to donate sperm/eggs within 6 months after signing the informed consent form.

22. Subjects or their spouses or partners who have plans for pregnancy within 6 months from the date of signing the informed consent form or who do not agree to use acceptable and effective contraceptive methods.

23. Patients deemed unsuitable for participation in the clinical trial by the investigators.

Study duration (Trial procedures)

In this study, the longest study duration for each subject was approximately 22 weeks, including a screening period (2 weeks prior to dosing), a run-in period (4 weeks), and a double-blind treatment period (16 weeks).

1. Screening period (Day -43 to Day -30 or Day -15 to Day -2)

After signing the written informed consent form, each potential subject underwent at least one screening visit to complete assessment of screening period: collecting medical history, medication history, demographic data, vital signs, physical examination, and laboratory tests at the research center (complete blood count, urinalysis, blood biochemistry, blood pregnancy test (for women of childbearing age only), pre-transfusion infectious disease screening, thyroid function, coagulation function), abdominal ultrasound, chest posteroanterior X-ray examination, and 12-lead electrocardiogram. Any test results outside the normal range were evaluated by the investigating physician to determine clinical significance of these results. The investigator needs to determine whether subjects have been receiving atorvastatin regularly for at least 4 weeks prior to screening; the subjects who did not meet this criterion entered the run-in period via Pathway 1, while the subjects who met this criterion entered the double-blind treatment period directly via Pathway 2.

2. Run-in period (Day -29 to Day -30): applicable only to subjects enrolled via Pathway 1

For subjects enrolled via Pathway 1, the investigator assessed whether the subjects met the criteria for entering the atorvastatin run-in period and determined whether the subjects complied with the protocol requirements and were suitable to begin taking the medication for run-in period.

In Pathway 1, subjects who had been receiving other statins at a stable dose prior to screening (excluding atorvastatin) and were deemed eligible for drug switching treatment by the investigator entered the run-in period and received an appropriate dose of atorvastatin for 4 weeks; and subjects who had been receiving atorvastatin prior to screening but failed to meet the requirement of regular dosing for 4 weeks could continue their current dose for another 4 weeks.

After 28 days of the run-in period, subjects were followed up and evaluated to determine their eligibility for the randomized treatment period.

3. Double-blind treatment period

Subjects enrolled via Pathway 1 and Pathway 2 who satisfied the criteria for entering the double-blind treatment period were randomized at a ratio of 5:5:5:3 to receive 140 mg of Antibody A, 420 mg of Antibody A, 420 mg of Evolocumab, and placebo, respectively, and continued treatment for 16 weeks. Specifically:

Antibody A 140 mg and Evolocumab 420 mg groups: Designed for dosing every other week on a background of atorvastatin therapy (with four doses given on Day 1, Day 29, Day 57, and Day 85);

Antibody A 420 mg group: Designed for dosing once every 8 weeks (420 mg of Antibody A administered on Day 1 and Day 57; and placebo administered on Day 29 and Day 85) on a background of atorvastatin therapy; and

Placebo group: Designed for placebo dosing every 4 weeks (placebo administered four times on Day 1, Day 29, Day 57, and Day 85) on a background of atorvastatin therapy.

Blood samples were collected from the subjects at predefined time points on Day 1 (first dosing) and Day 57 for pharmacokinetic testing. After 16 weeks of treatment, changes in lipid profile from baseline were evaluated and compared among groups.

Results overview:

Medication adherence: The investigational drug in this study was an injectable formulation, administered by a study nurse at each visit. Medication adherence was well controlled across all groups. Specifically, the Antibody A 140 mg group and the Evolocumab 420 mg group received dosing once every 4 weeks, while the Antibody A 420 mg group received dosing once every 8 weeks. All subjects completed dosing according to the dosing regimens.

All subjects in this study received atorvastatin as a background medication. In the Antibody A 140 mg group, Antibody A 420 mg group, and Evolocumab 420 mg group, 4, 2, and 2 subjects, respectively, had adherence rates below 80%, while all other subjects in these dosage groups had adherence rates $\geq 80\%$; and all subjects in the placebo group had adherence rates $\geq 80\%$.

Regarding the safety, based on the analysis of safety indicators (including adverse events, serious adverse events (SAEs), laboratory tests, vital signs, physical examinations, 12-lead electrocardiogram, immunogenicity), no significant differences in terms of the safety were observed between the Antibody A 140 mg group, the Antibody A 420 mg group, and the Evolocumab 420 mg group or the placebo group, indicating that both doses of Antibody A exhibited a favorable safety profile.

**Analysis of pharmacokinetic parameters**

Concentrations of free drug in serum were determined using a validated method. The results are shown in Table 5.

Table 5 shows the pharmacokinetic parameter sets (PKPS, N=20) of Antibody A after single and multiple dosing

| - | Single dosing (Antibody A) | | Multiple dosing (Antibody A) | |
|---|---|---|---|---|
| Dose | 140 mg | 420 mg | 140 mg | 420 mg |
| $T_{max}$(h) | 100.25 | 128.91 | 107.31 | 119.29 |
| $t_{1/2}$(h) | 135.32 | 189.94 | 161.64 | 192.75 |
| $C_{max}$(ng.mL$^{-1}$) | 13488.68 | 35861.39 | 14109.50 | 35052.27 |
| $AUC_{0-t}$(ng.h.mL$^{-1}$) | 4301250.19 | 14628771.49 | 5030409.54 | 14524760.80 |
| $AUC_{0-inf}$(ng.h.mL$^{-1}$) | 4589581.75 | 14434188.56 | 5608881.85 | 15957475.37 |

As shown in Table 5, at the same dose, the maximum plasma concentration and the time to reach the maximum plasma concentration of Antibody A were similar after single and multiple dosing, and no accumulation of Antibody A was observed following multiple dosing. The pharmacokinetic characteristics of Antibody A after single and multiple dosing were similar: the systemic exposure to Antibody A increased with increasing dose, and the half-life showed a certain degree of dose dependency. At both the 140 mg and 420 mg doses, the exposure to Antibody A was higher than that of Evolocumab.

**Lipid profile efficacy endpoints**

After 16 weeks of double-blind treatment, percentage changes from baseline in lipid profile were evaluated, and the results are shown in Table 6.

Table 6 presents a summary analysis of percentage changes from baseline in lipid profile after treatment of the subjects

| Percentage changes from baseline in each indicator after 16 weeks of treatment | Antibody A140 mg (N=10) | Antibody A 420 mg (N=10) | Evolocumab 420 mg (N=10) | Placebo group (N=6) |
|---|---|---|---|---|
| LDL-C | -73.34% | -57.47% | -69.43% | -28.35% |
| (non-HDL-C) | -69.82% | -51.41% | -64.01% | -22.51% |
| Total cholesterol | -52.87% | -38.54% | -47.02% | -16.50% |
| Apolipoprotein | -64.38% | -49.13% | -61.49% | -21.91% |
| Lipoprotein | -26.09% | -27.61% | -48.77% | -3.11% |
| Triglycerides | -36.44% | -16.75% | -24.63% | 28.80% |
| (sdLDL-C) | -67.69% | -55.80% | -59.66% | -27.53% |
| In the table above, non-HDL-C refers to non-high-density lipoprotein cholesterol, and sdLDL-C refers to small dense low-density lipoprotein cholesterol. | | | | |

As shown in Table 6, the percentage changes from baseline in lipid profile in the Antibody A 140 mg group (dosing once every 4 weeks) and the Antibody A 420 mg group (dosing once every 8 weeks) were comparable to those in the Evolocumab 420 mg group (dosing once every 4 weeks), and were significantly greater than those in the placebo group. These results indicate that Antibody A supports less frequent dosing or a lower dose at the same dosing frequency.
The above examples are preferred implementations of the present invention but do not limit implementations of the present invention. Any other changes, modifications, substitutions, combinations and simplifications made without departing from the spirit and principle of the present invention shall be equivalent replacement methods, and fall within the scope of protection of the present invention.

**Claims**

1. A method for treating or preventing cholesterol-related diseases, comprising:
administering 35-420 mg of a PCSK9 antibody to a subject, wherein a heavy chain variable region of the PCSK9 antibody comprises an HCDR1 as shown in SEQ ID NO:1, an HCDR2 as shown in SEQ ID NO:2, and an HCDR3 as shown in SEQ ID NO:3; and a light chain variable region of the PCSK9 antibody comprises an LCDR1 as shown in SEQ ID NO:7, an LCDR2 as shown in SEQ ID NO:8, and an LCDR3 as shown in SEQ ID NO:9.

2. The method according to claim 1, wherein 70 mg, 140 mg, 210 mg, 280 mg, 350 mg, or 420 mg of the PCSK9 antibody is administered to the subject.

3. The method according to claim 1, wherein 70 mg of the PCSK9 antibody is administered to the subject once every week or once every two weeks, or 140 mg of the PCSK9 antibody is administered to the subject once every two weeks, once every four weeks, once every six weeks, or once every eight weeks, or 280 mg of the PCSK9 antibody is administered to the subject once every two weeks, once every four weeks, once every six weeks, or once every eight weeks, or 420 mg of the PCSK9 antibody is administered to the subject once every four weeks, once every six weeks, once every eight weeks, or once every twelve weeks.

4. The method according to any one of claims 1-3, wherein an administration route is subcutaneous injection or intramuscular injection.

5. The method according to any one of claims 1-3, wherein 1-16 weeks after 70-420 mg of the PCSK9 antibody is administered to the subject, an area under the plasma concentration-time curve from zero to the time of the last quantifiable concentration ($AUC_{0-t}$) in the subject is at least greater than 5,000,000 ng•h/mL.

6. The method according to any one of claims 1-3, wherein after 140-420 mg of the PCSK9 antibody is administered to the subject, a reduction of low-density lipoprotein cholesterol (LDL-C) by at least 50% is maintained for 25-60 days.

7. A method for treating or preventing cholesterol-related diseases, comprising:

   administering a clinical dose of a statin drug and 35-420 mg of a PCSK9 antibody to a subject; wherein a heavy chain variable region of the PCSK9 antibody comprises an HCDR1 as shown in SEQ ID NO:1, an HCDR2 as shown in SEQ ID NO:2, and an HCDR3 as shown in SEQ ID NO:3; and a light chain variable region of the PCSK9 antibody comprises an LCDR1 as shown in SEQ ID NO:7, an LCDR2 as shown in SEQ ID NO:8, and an LCDR3 as shown in SEQ ID NO:9.

8. The method according to claim 7, wherein 70 mg, 140 mg, 210 mg, 280 mg, 350 mg, or 420 mg of the PCSK9 antibody is administered to the subject.

9. The method according to claim 7, wherein 70 mg of the PCSK9 antibody is administered to the subject once every week or once every two weeks, or 140 mg of the PCSK9 antibody is administered to the subject once every two weeks, once every four weeks, once every six weeks, or once every eight weeks, or 280 mg of the PCSK9 antibody is administered to the subject once every two weeks, once every four weeks, once every six weeks, or once every eight weeks, or 420 mg of the PCSK9 antibody is administered to the subject once every four weeks, once every six weeks, once every eight weeks, or once every twelve weeks.

10. The method according to any one of claims 7-9, wherein the PCSK9 antibody is administered by subcutaneous injection or intramuscular injection, and the statin drug is administered orally.

11. The method according to any one of claims 7-9, wherein the statin drug is selected from atorvastatin, cerivastatin, fluvastatin, lovastatin, mevastatin, pitavastatin, pravastatin, rosuvastatin, and simvastatin; and when the statin drug is atorvastatin, the clinical dose is 10-80 mg, and preferably 20 mg or 30 mg.

12. The method according to any one of claims 7-9, wherein 1-16 weeks after 70-420 mg of the PCSK9 antibody is administered to the subject, an $AUC_{0-t}$ in the subject is at least greater than 4,000,000 ng•h/mL.

## INTERNATIONAL SEARCH REPORT

| International application No. |
| --- |
| **PCT/CN2024/111651** |

**A.    CLASSIFICATION OF SUBJECT MATTER**

C07K16/40(2006.01)i; A61K39/395(2006.01)i; C07K16/38(2006.01)i; A61P3/06(2006.01)i; A61P9/00(2006.01)i

According to International Patent Classification (IPC) or to both national classification and IPC

**B.    FIELDS SEARCHED**

Minimum documentation searched (classification system followed by classification symbols)

IPC: C07K, A61K, A61P

Documentation searched other than minimum documentation to the extent that such documents are included in the fields searched

Electronic data base consulted during the international search (name of data base and, where practicable, search terms used)

VEN, CNABS, PubMed, DWPI, CNTXT, WOTXT, USTXT, EPTXT, JPTXT, CNKI, Web of Science, 百度学术, Baidu Scholar, Patentics, 中国生物序列检索系统, China Biological Sequence Search System, NCBI Genbank, EBI, STNext: 申请人, Applicants, 发明人, Inventors, 序列1-3, 7-9, 13, 14, sequences 1-3, 7-9, 13 and 14, 高脂血, 胆固醇, 低密度脂蛋白胆固醇, 抗体, 前蛋白转化酶枯草杆菌蛋白酶, PCSK9, low-densitylipoprotein cholesterol, LDL-C, proprotein convertasesubtilisin/kexin type 9, antibody, hyperlipidemia, cholesterol

**C.    DOCUMENTS CONSIDERED TO BE RELEVANT**

| Category* | Citation of document, with indication, where appropriate, of the relevant passages | Relevant to claim No. |
| --- | --- | --- |
| X | CN 107840893 A (WUXI BIOLOGICS (SHANGHAI) CO., LTD.) 27 March 2018 (2018-03-27)<br>     abstract, claims 1-18, and description, paragraphs 251, 262, 272 and 367, and sequences 77 and 79 | 1-12 |
| X | WO 2018054241 A1 (WUXI BIOLOGICS (SHANGHAI) CO., LTD.) 29 March 2018 (2018-03-29)<br>     abstract, claims 1-37, and sequences 77 and 79 | 1-12 |
| A | CN 106062003 A (SANOFI BIOTECHNOLOGY INC. et al.) 26 October 2016 (2016-10-26)<br>     claims 1-11 | 1-12 |
| A | CN 114423866 A (INNOVENT BIOLOGICS (SUZHOU) CO., LTD.) 29 April 2022 (2022-04-29)<br>     claims 1-27 | 1-12 |

| ✓ Further documents are listed in the continuation of Box C. | ✓ See patent family annex. |
| --- | --- |

|   |   |   |   |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **14 November 2024** | **20 November 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/111651** |

### C.   DOCUMENTS CONSIDERED TO BE RELEVANT

| Category* | Citation of document, with indication, where appropriate, of the relevant passages | Relevant to claim No. |
| --- | --- | --- |
| A | US 2009142352 A1 (JACKSON SIMON MARK et al.) 04 June 2009 (2009-06-04)<br>claims 1-10 | 1-12 |
| A | WO 2013166448 A1 (AMGEN INC.) 07 November 2013 (2013-11-07)<br>abstract, and claims 1-18 | 1-12 |
| A | WO 2017200715 A1 (CYMABAY THERAPEUTICS, INC.) 23 November 2017 (2017-11-23)<br>claims 1-20 | 1-12 |
| A | 柯元南 (KE, Yuannan). "一类新的降脂药——PCSK9抑制剂 (Non-official translation: A New Lipid-Lowering Drug—PCSK9 Inhibitors)"<br>中老年保健 (Non-official translation: Health for the Elderly and Middle-Aged).<br>No. no. 6, 31 December 2020 (2020-12-31),<br>pp. 26-27 | 1-12 |

Form PCT/ISA/210 (second sheet) (July 2022)

**INTERNATIONAL SEARCH REPORT**

| International application No. |
| --- |
| **PCT/CN2024/111651** |

| **Box No. II** **Observations where certain claims were found unsearchable (Continuation of item 2 of first sheet)** |
| --- |

This international search report has not been established in respect of certain claims under Article 17(2)(a) for the following reasons:

1. ☑ Claims Nos.: **1-12**
   because they relate to subject matter not required to be searched by this Authority, namely:

   Claims 1-12 relate to a method for treating or preventing cholesterol-related diseases, which falls within the scope defined in PCT Rule 39.1(iv) for which no search is required. However, a search is still carried out on the basis of the pharmaceutical use thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| | International application No. |
|---|---|
| | **PCT/CN2024/111651** |

| Patent document cited in search report | | | Publication date (day/month/year) | Patent family member(s) | | | Publication date (day/month/year) |
|---|---|---|---|---|---|---|---|
| CN | 107840893 | A | 27 March 2018 | TW | 201825518 | A | 16 July 2018 |
| | | | | TWI | 782920 | B | 11 November 2022 |
| | | | | HK | 1249538 | A1 | 02 November 2018 |
| WO | 2018054241 | A1 | 29 March 2018 | KR | 20190049866 | A | 09 May 2019 |
| | | | | EP | 3515950 | A1 | 31 July 2019 |
| | | | | EP | 3515950 | A4 | 28 October 2020 |
| | | | | US | 2019233542 | A1 | 01 August 2019 |
| | | | | US | 11111313 | B2 | 07 September 2021 |
| | | | | JP | 2020502991 | A | 30 January 2020 |
| CN | 106062003 | A | 26 October 2016 | EA | 201690889 | A1 | 30 November 2016 |
| | | | | US | 2020024364 | A1 | 23 January 2020 |
| | | | | CA | 2929778 | A1 | 21 May 2015 |
| | | | | EP | 3882273 | A1 | 22 September 2021 |
| | | | | TW | 201609132 | A | 16 March 2016 |
| | | | | TWI | 670077 | B | 01 September 2019 |
| | | | | US | 2023406957 | A1 | 21 December 2023 |
| | | | | WO | 2015073494 | A1 | 21 May 2015 |
| | | | | WO | 2015073494 | A8 | 19 May 2016 |
| | | | | EP | 3068803 | A1 | 21 September 2016 |
| | | | | EP | 3068803 | B1 | 20 January 2021 |
| | | | | KR | 20160081978 | A | 08 July 2016 |
| | | | | JP | 2016538277 | A | 08 December 2016 |
| | | | | JP | 6616298 | B2 | 04 December 2019 |
| | | | | AU | 2014348765 | A1 | 09 June 2016 |
| | | | | IL | 245564 | A0 | 30 June 2016 |
| | | | | IL | 245564 | B | 31 May 2020 |
| | | | | US | 2015152191 | A1 | 04 June 2015 |
| | | | | US | 10428157 | B2 | 01 October 2019 |
| | | | | MX | 2016006226 | A | 07 September 2016 |
| CN | 114423866 | A | 29 April 2022 | WO | 2021052472 | A1 | 25 March 2021 |
| | | | | KR | 20220064998 | A | 19 May 2022 |
| | | | | TW | 202124454 | A | 01 July 2021 |
| | | | | TWI | 759867 | B | 01 April 2022 |
| | | | | BR | 112022005060 | A2 | 21 June 2022 |
| | | | | JP | 2022548950 | A | 22 November 2022 |
| | | | | AU | 2020347729 | A1 | 31 March 2022 |
| | | | | CA | 3155065 | A1 | 25 March 2021 |
| | | | | EP | 4063506 | A1 | 28 September 2022 |
| | | | | EP | 4063506 | A4 | 06 September 2023 |
| | | | | US | 2022332846 | A1 | 20 October 2022 |
| US | 2009142352 | A1 | 04 June 2009 | EP | 3666797 | A1 | 17 June 2020 |
| | | | | EP | 3666797 | B1 | 17 May 2023 |
| | | | | PL | 3666797 | T3 | 28 August 2023 |
| | | | | TW | 201716444 | A | 16 May 2017 |
| | | | | TWI | 633122 | B | 21 August 2018 |
| | | | | US | 2011027287 | A1 | 03 February 2011 |
| | | | | PE | 20180173 | A1 | 22 January 2018 |
| | | | | DK | 3666797 | T3 | 30 May 2023 |
| | | | | DK | 3666797 | T5 | 26 August 2024 |
| | | | | IL | 204013 | A | 31 July 2017 |

Form PCT/ISA/210 (patent family annex) (July 2022)

<table>
<tr><td colspan="2" align="center"><strong>INTERNATIONAL SEARCH REPORT</strong><br>Information on patent family members</td><td colspan="2">International application No.<br><strong>PCT/CN2024/111651</strong></td></tr>
<tr><td align="center">Patent document<br>cited in search report</td><td align="center">Publication date<br>(day/month/year)</td><td align="center">Patent family member(s)</td><td align="center">Publication date<br>(day/month/year)</td></tr>
<tr><td></td><td></td><td>PE   20131400  A1</td><td>16 December 2013</td></tr>
<tr><td></td><td></td><td>US  2012020975  A1</td><td>26 January 2012</td></tr>
<tr><td></td><td></td><td>US   9045547  B2</td><td>02 June 2015</td></tr>
<tr><td></td><td></td><td>RS    54756  B1</td><td>31 October 2016</td></tr>
<tr><td></td><td></td><td>RS    54756  B2</td><td>31 July 2024</td></tr>
<tr><td></td><td></td><td>US  2014357854  A1</td><td>04 December 2014</td></tr>
<tr><td></td><td></td><td>HUS   1600029  I1</td><td>28 September 2016</td></tr>
<tr><td></td><td></td><td>CA   2696252  A1</td><td>26 February 2009</td></tr>
<tr><td></td><td></td><td>CA   2696252  C</td><td>14 June 2016</td></tr>
<tr><td></td><td></td><td>DK   2215124  T3</td><td>30 May 2016</td></tr>
<tr><td></td><td></td><td>DK   2215124  T4</td><td>29 January 2024</td></tr>
<tr><td></td><td></td><td>PL   2215124  T3</td><td>31 August 2016</td></tr>
<tr><td></td><td></td><td>PL   2215124  T5</td><td>20 November 2023</td></tr>
<tr><td></td><td></td><td>TW  202330625  A</td><td>01 August 2023</td></tr>
<tr><td></td><td></td><td>US  2012093818  A1</td><td>19 April 2012</td></tr>
<tr><td></td><td></td><td>US   9920134  B2</td><td>20 March 2018</td></tr>
<tr><td></td><td></td><td>EA  201000356  A1</td><td>30 August 2010</td></tr>
<tr><td></td><td></td><td>EA    032106  B1</td><td>30 April 2019</td></tr>
<tr><td></td><td></td><td>HRP  20230503  T3</td><td>15 September 2023</td></tr>
<tr><td></td><td></td><td>CY   2016029  I1</td><td>26 April 2017</td></tr>
<tr><td></td><td></td><td>JP  2023071833  A</td><td>23 May 2023</td></tr>
<tr><td></td><td></td><td>PE   20140014  A1</td><td>31 January 2014</td></tr>
<tr><td></td><td></td><td>PE   20091006  A1</td><td>14 August 2009</td></tr>
<tr><td></td><td></td><td>MX  2020008898  A</td><td>12 October 2020</td></tr>
<tr><td></td><td></td><td>US  2013085265  A1</td><td>04 April 2013</td></tr>
<tr><td></td><td></td><td>US  2014357852  A1</td><td>04 December 2014</td></tr>
<tr><td></td><td></td><td>US   8981064  B2</td><td>17 March 2015</td></tr>
<tr><td></td><td></td><td>US  2014357851  A1</td><td>04 December 2014</td></tr>
<tr><td></td><td></td><td>US   9493576  B2</td><td>15 November 2016</td></tr>
<tr><td></td><td></td><td>KR  20170012592  A</td><td>02 February 2017</td></tr>
<tr><td></td><td></td><td>US  2013052201  A1</td><td>28 February 2013</td></tr>
<tr><td></td><td></td><td>US  2012213797  A1</td><td>23 August 2012</td></tr>
<tr><td></td><td></td><td>DE  202008018562  U1</td><td>02 November 2015</td></tr>
<tr><td></td><td></td><td>JP  2010536384  A</td><td>02 December 2010</td></tr>
<tr><td></td><td></td><td>JP   5441905  B2</td><td>12 March 2014</td></tr>
<tr><td></td><td></td><td>PE   20221507  A1</td><td>04 October 2022</td></tr>
<tr><td></td><td></td><td>CY   2016023  I1</td><td>26 April 2017</td></tr>
<tr><td></td><td></td><td>NZ    584101  A</td><td>28 September 2012</td></tr>
<tr><td></td><td></td><td>US  2014357853  A1</td><td>04 December 2014</td></tr>
<tr><td></td><td></td><td>US   9056915  B2</td><td>16 June 2015</td></tr>
<tr><td></td><td></td><td>KR  20100057070  A</td><td>28 May 2010</td></tr>
<tr><td></td><td></td><td>KR   101494932  B1</td><td>26 February 2015</td></tr>
<tr><td></td><td></td><td>KR  20200074262  A</td><td>24 June 2020</td></tr>
<tr><td></td><td></td><td>US  2013079501  A1</td><td>28 March 2013</td></tr>
<tr><td></td><td></td><td>JP  2015166345  A</td><td>24 September 2015</td></tr>
<tr><td></td><td></td><td>JP   5906333  B2</td><td>20 April 2016</td></tr>
<tr><td></td><td></td><td>TW  201500375  A</td><td>01 January 2015</td></tr>
<tr><td></td><td></td><td>TWI   675847  B</td><td>01 November 2019</td></tr>
<tr><td></td><td></td><td>KR  20240113594  A</td><td>22 July 2024</td></tr>
<tr><td></td><td></td><td>WO  2009026558  A1</td><td>26 February 2009</td></tr>
</table>

Form PCT/ISA/210 (patent family annex) (July 2022)

| Patent document cited in search report | Publication date (day/month/year) | Patent family member(s) | | | Publication date (day/month/year) |
|---|---|---|---|---|---|
| | | JP | 2018118974 | A | 02 August 2018 |
| | | JP | 6638009 | B2 | 29 January 2020 |
| | | IL | 304868 | A | 01 October 2023 |
| | | TW | 200916481 | A | 16 April 2009 |
| | | TWI | 441833 | B | 21 June 2014 |
| | | SI | 3666797 | T1 | 30 October 2023 |
| | | FR | 16002511 | | 08 July 2016 |
| | | FR | 16002512 | | 08 June 2018 |
| | | BR | 122018012430 | B1 | 08 October 2019 |
| | | BR | 122018012430 | B8 | 27 July 2021 |
| | | US | 2014235831 | A1 | 21 August 2014 |
| | | US | 8889834 | B2 | 18 November 2014 |
| | | PH | 12013502286 | A1 | 22 February 2016 |
| | | PE | 20140221 | A1 | 21 February 2014 |
| | | KR | 20210028741 | A | 12 March 2021 |
| | | NO | 2016015 | I1 | 12 August 2016 |
| | | HUS | 1600036 | I1 | 28 October 2016 |
| | | US | 8030457 | B2 | 04 October 2011 |
| | | HK | 1143824 | A1 | 14 January 2011 |
| | | KR | 20220031734 | A | 11 March 2022 |
| | | US | 2012251544 | A1 | 04 October 2012 |
| | | LTPA | 2016021 | I1 | 27 June 2016 |
| | | LTC | 2215124 | I2 | 25 April 2023 |
| | | SG | 184702 | A1 | 30 October 2012 |
| | | SI | 2215124 | T1 | 30 September 2016 |
| | | AR | 068011 | A1 | 28 October 2009 |
| | | DE | 19207796 | T1 | 10 March 2022 |
| | | DE | 19207796 | T9 | 23 February 2023 |
| | | MA | 31978 | B1 | 03 January 2011 |
| | | KR | 20230080496 | A | 07 June 2023 |
| | | HRP | 20160494 | T1 | 03 June 2016 |
| | | HRP | 20160494 | T4 | 27 October 2023 |
| | | NO | 2023012 | I1 | 20 March 2023 |
| | | US | 2014228547 | A1 | 14 August 2014 |
| | | US | 8883983 | B2 | 11 November 2014 |
| | | US | 2013058944 | A1 | 07 March 2013 |
| | | LTPA | 2016025 | I1 | 12 September 2016 |
| | | ES | 2917423 | T3 | 08 July 2022 |
| | | SI | 2215124 | T1 | 30 September 2016 |
| | | SI | 2215124 | T2 | 30 November 2023 |
| | | KR | 20140064962 | A | 28 May 2014 |
| | | KR | 101702194 | B1 | 03 February 2017 |
| | | US | 2014228557 | A1 | 14 August 2014 |
| | | US | 8871914 | B2 | 28 October 2014 |
| | | US | 2013079502 | A1 | 28 March 2013 |
| | | CY | 1117940 | T1 | 26 April 2017 |
| | | KR | 20180035947 | A | 06 April 2018 |
| | | BRPI | 0816117 | A2 | 10 March 2015 |
| | | BRPI | 0816117 | B1 | 18 December 2018 |
| | | BRPI | 0816117 | B8 | 25 May 2021 |

Form PCT/ISA/210 (patent family annex) (July 2022)

<table>
<tr><td colspan="2"><strong>INTERNATIONAL SEARCH REPORT</strong><br>Information on patent family members</td><td colspan="2">International application No.<br><strong>PCT/CN2024/111651</strong></td></tr>
</table>

| Patent document cited in search report | Publication date (day/month/year) | Patent family member(s) | | Publication date (day/month/year) |
|---|---|---|---|---|
| | | MX | 2010001921 A | 11 March 2010 |
| | | US | 2014235830 A1 | 21 August 2014 |
| | | US | 8871913 B2 | 28 October 2014 |
| | | LU | 93180 I2 | 18 October 2016 |
| | | JP | 2020058376 A | 16 April 2020 |
| | | PT | 3666797 T | 07 June 2023 |
| | | LU | 93096 I2 | 01 August 2016 |
| | | JOP | 20080381 B1 | 28 March 2023 |
| | | SG | 10201710183 TA | 30 January 2018 |
| | | US | 2015087819 A1 | 26 March 2015 |
| | | NL | 300818 I2 | 14 July 2016 |
| | | LT | 3666797 T | 10 August 2023 |
| | | MY | 180102 A | 22 November 2020 |
| | | RS | 64295 B1 | 31 July 2023 |
| | | US | 2013245235 A1 | 19 September 2013 |
| | | US | 8829165 B2 | 09 September 2014 |
| | | JP | 2016182114 A | 20 October 2016 |
| | | TW | 201906871 A | 16 February 2019 |
| | | TWI | 799416 B | 21 April 2023 |
| | | FI | 2215124 T4 | 10 October 2023 |
| | | US | 2009326202 A1 | 31 December 2009 |
| | | US | 8563698 B2 | 22 October 2013 |
| | | EP | 3202791 A1 | 09 August 2017 |
| | | FI | 3666797 T3 | 25 May 2023 |
| | | NO | 2016010 I1 | 03 June 2016 |
| | | TW | 201500374 A | 01 January 2015 |
| | | TWI | 675846 B | 01 November 2019 |
| | | HUE | 028162 T2 | 28 December 2016 |
| | | TW | 201500376 A | 01 January 2015 |
| | | TWI | 675848 B | 01 November 2019 |
| | | IL | 252616 A0 | 31 July 2017 |
| | | IL | 252616 B | 31 May 2020 |
| | | US | 2015031870 A1 | 29 January 2015 |
| | | HUE | 062493 T2 | 28 November 2023 |
| | | CO | 6230997 A2 | 20 December 2010 |
| | | PE | 20140220 A1 | 21 February 2014 |
| | | US | 2012020976 A1 | 26 January 2012 |
| | | US | 8168762 B2 | 01 May 2012 |
| | | PH | 12013502285 A1 | 08 August 2016 |
| | | UA | 127402 C2 | 16 August 2023 |
| | | TN | 2010000063 A1 | 26 September 2011 |
| | | CL | 2008002495 A1 | 04 September 2009 |
| | | ES | 2573258 T3 | 06 June 2016 |
| | | ES | 2573258 T5 | 23 February 2024 |
| | | PE | 20140231 A1 | 08 March 2014 |
| | | IL | 273353 A | 31 May 2020 |
| | | IL | 273353 B1 | 01 September 2023 |
| | | IL | 273353 B2 | 01 January 2024 |
| | | ES | 2946083 T3 | 12 July 2023 |
| | | AU | 2008288791 A1 | 26 February 2009 |

Form PCT/ISA/210 (patent family annex) (July 2022)

INTERNATIONAL SEARCH REPORT
Information on patent family members

| International application No. |
| --- |
| **PCT/CN2024/111651** |

| Patent document cited in search report | | | Publication date (day/month/year) | Patent family member(s) | | | Publication date (day/month/year) |
| --- | --- | --- | --- | --- | --- | --- | --- |
| | | | | AU | 2008288791 | B2 | 30 October 2014 |
| | | | | JP | 2014043446 | A | 13 March 2014 |
| | | | | JP | 5705288 | B2 | 22 April 2015 |
| | | | | CR | 11328 | A | 12 July 2010 |
| | | | | US | 2014228545 | A1 | 14 August 2014 |
| | | | | US | 8859741 | B2 | 14 October 2014 |
| | | | | US | 2014357850 | A1 | 04 December 2014 |
| | | | | US | 2012027765 | A1 | 02 February 2012 |
| | | | | EP | 2215124 | A1 | 11 August 2010 |
| | | | | EP | 2215124 | B1 | 24 February 2016 |
| | | | | EP | 2215124 | B2 | 19 July 2023 |
| | | | | EP | 2215124 | B9 | 27 December 2023 |
| WO | 2013166448 | A1 | 07 November 2013 | EA | 201792336 | A2 | 29 June 2018 |
| | | | | EA | 201792336 | A3 | 30 November 2018 |
| | | | | EA | 039663 | B1 | 24 February 2022 |
| | | | | ME | 03797 | B | 20 April 2021 |
| | | | | EP | 3656399 | A1 | 27 May 2020 |
| | | | | RS | 60794 | B1 | 30 October 2020 |
| | | | | WO | 2013166448 | A8 | 19 June 2014 |
| | | | | CY | 1123250 | T1 | 29 October 2021 |
| | | | | HK | 1207592 | A1 | 05 February 2016 |
| | | | | SI | 2844285 | T1 | 30 November 2020 |
| | | | | EP | 2844285 | A1 | 11 March 2015 |
| | | | | EP | 2844285 | B1 | 15 July 2020 |
| | | | | ES | 2810907 | T3 | 09 March 2021 |
| | | | | HUE | 050276 | T2 | 30 November 2020 |
| | | | | HRP | 20201268 | T1 | 19 February 2021 |
| | | | | TN | 2013000467 | A1 | 30 March 2015 |
| | | | | DK | 2844285 | T3 | 10 August 2020 |
| | | | | PL | 2844285 | T3 | 28 December 2020 |
| | | | | US | 2014030270 | A1 | 30 January 2014 |
| | | | | LT | 2844285 | T | 12 October 2020 |
| | | | | PT | 2844285 | T | 25 August 2020 |
| WO | 2017200715 | A1 | 23 November 2017 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021243002 A

- CN 201710816808